# EUROPEAN PATENT APPLICATION

(11) **EP 4 066 814 A1**
(43) Date of publication of application: **05.10.2022**
(21) Application number: 21908070.2
(22) Date of filing: 22.11.2021
(51) Int. Cl.: A61K 8/69

(54) **MODIFIED PEARLESCENT POWDER AND PREPARATION METHOD THEREFOR, AND COSMETIC**

(30) Priority: 05.02.2021 CN 202110162074
(71) Applicant: Shanghai Co-fun Biotech Co., Ltd., Shanghai 201508 (CN)
(72) Inventor: XIE, Xu, Shanghai 201508 (CN); QIN, Haifeng, Shanghai 201508 (CN); WU, Jianjun, Shanghai 201508 (CN); YANG, Xiaojuan, Shanghai 201508 (CN)
(74) Representative: Petraz, Gilberto Luigi
(86) International application number: PCT/CN2021/132080
(87) International publication number: WO 2022/166314

(57) **Abstract**

The present disclosure relates to a modified pearlescent powder and a method of preparing the same. The modified pearlescent powder includes a pearlescent powder, a lauroyl lysine powder covered on a surface of the pearlescent powder, and a fluorine-containing silane coupling agent connected to the pearlescent powder by covalent bonds. The present disclosure also relates to a cosmetic including the modified pearlescent powder.

## Description

### CROSS-REFERENCE TO RELATED APPLICATION

This application claims all benefits accruing from China Patent Application No. 202110162074.5, filed on February 5, 2021, in the China National Intellectual Property Administration, and titled "MODIFIED PEARLESCENT POWDER, METHOD OF PREPARING THE SAME, AND COSMETICS INCLUDING THE SAME" the content of which is hereby incorporated by reference.

### TECHNICAL FIELD

The present disclosure relates to a technical field of cosmetics, in particular to a modified pearlescent powder, a method of preparing the same, and cosmetics including the same.

### BACKGROUND

Pearlescent powder is widely applied in the cosmetics industry because of its unique gloss, and it is an important colorant in beauty cosmetics for face, lips, eyes, and nails. However, conventional pearlescent powders have several defects, for example, pearlescent powders with a particle size of less than or equal to 60 microns are not shiny enough, while those with a particle size greater than 60 microns are not skin-adhesive and soft enough. In addition, since the pearlescent powders are hydrophilic and lipophilic, conventional cosmetics are difficult to be applied evenly on skin and have poor makeup retention.

### SUMMARY

On such a basis, it is necessary to solve the problems and provide a modified pearlescent powder that is softer and more skin-adhesive, while having a hydrophobic and lipophobic effect, a method of preparing the same, and cosmetics employing the same.

A modified pearlescent powder includes a pearlescent powder, a lauroyl lysine powder covered on a surface of the pearlescent powder, and a fluorine-containing silane coupling agent connected to the pearlescent powder by covalent bonds.

In one embodiment, the lauroyl lysine powder is in a flake-shaped structure.

In one embodiment, a part of the fluorine-containing silane coupling agent is associated with each other to form a network structure.

In one embodiment, a mass of the fluorine-containing silane coupling agent is in a range of 0.1% to 10.0% of a mass of the pearlescent powder, and a mass of the lauroyl lysine powder is in a range of 0.1% to 10.0% of the mass of the pearlescent powder.

In one embodiment, a general formula of the fluorine-containing silane coupling agent is as follows: R-Si-(OCₘH₂ₘ₊₁)₃. In the formula, R is defined as CₙF₂ₙ₊₁-(CH₂)₂-, wherein n is an integer which is greater than or equal to 1 and less than or equal to 6 and m is 1 or 2.

In one embodiment, the fluorine-containing silane coupling agent includes at least one of perfluorooctyltriethoxysilane, tridecafluorooctyltrimethoxysilane, or heptadecafluorodecyltrimethoxysilane.

A method for preparing the modified pearlescent powder as described above includes: mixing the pearlescent powder, the fluorine-containing silane coupling agent and the lauroyl lysine powder to obtain a mixture, and heating the mixture to obtain the modified pearlescent powder.

In one embodiment, a temperature of the heating is in a range of 100 degrees centigrade to 120 degrees centigrade, and a time of the heating is in a range of 1hour to 3 hours.

A cosmetic includes the modified pearlescent powder as described above.

In one embodiment, the modified pearlescent powder is directionally distributed in the cosmetic.

In the present disclosure, a pearlescent powder is chemically and physically modified with a fluorine-containing silane coupling agent and a lauroyl lysine powder, so that a modified pearlescent powder thus obtained is softer and more skin-adhesive. Meanwhile, the modified pearlescent powder has an extremely low surface tension, and a hydrophobic and lipophobic effect. Therefore, the modified pearlescent powder may form a mirror effect in cosmetics, and show higher glossiness. At the same time, a spreadability of the cosmetics including the modified pearlescent powder may be effectively improved, so that the cosmetics can be spread more evenly on skin and has better makeup retention.

### BRIEF DESCRIPTION OF THE DRAWINGS

FIG. 1 is an SEM image of a lauroyl lysine powder covered on a surface of a pearlescent powder in a modified pearlescent powder obtained in Embodiment 1.
FIG. 2 is an effect image of dispersing the pearlescent powder and the obtained modified pearlescent powder of Embodiment 1 in glycerin.
FIG. 3 is an effect image of applying a modified pearlescent powder dispersed in glycerin and a pearlescent powder dispersed in glycerin on skin.
FIG. 4 is an effect image of dispersing the modified pearlescent powder obtained in Embodiment 1 in water.
FIG. 5 is an image showing an effect of applying an eye shadow obtained in Application Embodiment 1-1 and Application Comparative Embodiment 1-1 on skin.

### DETAILED DESCRIPTION

A modified pearlescent powder provided by the present disclosure, a method of preparing the same, and cosmetics will be further described hereinafter.

The modified pearlescent powder provided by the present disclosure can be mainly used in cosmetics to improve the glossiness, skin affinity and makeup retention of the cosmetics.

In one embodiment, the modified pearlescent powder can include a pearlescent powder, a lauroyl lysine powder covered on a surface of the pearlescent powder, and a fluorine-containing silane coupling agent connected to the pearlescent powder by covalent bonds.

The pearlescent powder can be in a layered structure which includes a substrate and a covering layer. The substrate can include at least one of a mica substrate, a silica substrate, an aluminum substrate, a glass substrate, an oxide substrate, a kaolin substrate, a ceramic substrate, or a graphite substrate. The covering layer can include at least one of a titanium dioxide covering layer, an iron oxide covering layer, a CI77510 (pigment blue 27, iron ferrocyanide) covering layer, or a carmine covering layer.

Due to different refractive indexes of the titanium dioxide and the mica, a better pearlescent effect, color effect and glittery effect can be produced by a multiple reflection and interference effects of light. In one embodiment, the pearlescent powder can be optionally a mica titanium pearlescent powder, wherein the substrate of the mica titanium pearlescent powder can be a mica substrate, and the covering layer thereof can be a titanium dioxide covering layer.

In the modified pearlescent powder of the present disclosure, a general formula of the fluorine-containing silane coupling agent can be R-Si-(OCₘH₂ₘ₊₁)₃. In the formula, R can be defined as CₙF₂ₙ₊₁-(CH₂)₂-, n can be an integer which can be greater than or equal to 1 and less than or equal to 6 and m can be 1 or 2. The fluorine-containing silane coupling agent connected to the pearlescent powder by the covalent bonds can be obtained by the following steps: hydrolyzing the alkoxy groups of the fluorine-containing silane coupling agent to form silanols; reacting the silanols with hydroxyl groups on the surface of the pearlescent powder to form hydrogen bonds; and dehydrating and condensing. In some embodiments, the fluorine-containing silane coupling agent connected to the pearlescent powder by the covalent bonds can be fluorine-containing segments, and the fluorine-containing segments can include at least one segment represented by Formula (1):

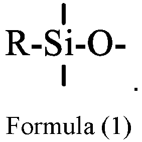

Due to a low surface energy of fluorine, when the surface of the pearlescent powder is connected with a fluorine-containing silane coupling agent via covalent bonds, a surface tension of the modified pearlescent powder can be extremely low, so that the modified pearlescent powder can have a hydrophobic and lipophobic effect.

In one embodiment, parts of the fluorine-containing silane coupling agent can be associated with each other to form a network structure which covers the surface of the pearlescent powder and forms a film layer.

In order to make the modified pearlescent powder have a hydrophobic and lipophobic effect, in one embodiment, a mass of the fluorine-containing silane coupling agent can be in a range of 0.1% to 10.0% of a mass of the pearlescent powder. In one embodiment, the mass of the fluorine-containing silane coupling agent can be in a range of 1.5% to 3% of that of the pearlescent powder.

In one embodiment, the fluorine-containing silane coupling agent can include at least one of perfluorooctyltriethoxysilane, tridecafluorooctyltrimethoxysilane, or heptadecafluorodecyltrimethoxysilane. In one embodiment, the fluorine-containing silane coupling agent can be perfluorooctyltriethoxysilane.

The lauroyl lysine powder can be an amino acid derivative, which has a soft and skin-adhesive effect. Therefore, when the lauroyl lysine powder is covered on the pearlescent powder, the modified pearlescent powder can have a softer and more skin-adhesive effect.

In order to make the modified pearlescent powder have a relatively better softness and skin-adhesive effect, in one embodiment, a mass of the lauroyl lysine powder can be in a range of 0.1% to 10.0% of that of the pearlescent powder. In one embodiment, the mass of the lauroyl lysine powder can be in a range of 3% to 7% of that of the pearlescent powder.

The lauroyl lysine powder can be coated on the surface of the pearlescent powder mainly by a physical effect of van der Waals forces. When parts of the fluorine-containing silane coupling agent is associated with each other to form a network structure, the network structure may also play a role of fixing the lauroyl lysine powder, so that the combination of the lauroyl lysine powder and the pearlescent powder can be more reliable.

In order to make the modified pearlescent powder have a softer and more skin-adhesive effect, in one embodiment, the lauroyl lysine powder can be in a flake-shaped structure.

Furthermore, in order to make the modified pearlescent powder have a better comprehensive performance including a better pearlescent effect, color effect and glittery effect, hydrophobic and lipophobic effect, and softness and skin-adhering effect, in one embodiment, a size of the lauroyl lysine powder in a flake-shaped structure is smaller than that of the pearlescent powder. In some embodiments, lauroyl lysine powder in the flake-shaped structure and the segments of the fluorine-containing silane coupling agent can be alternately distributed with each other.

Therefore, in the present disclosure, the pearlescent powder is modified by chemical and physical methods with the fluorine-containing silane coupling agent and the lauroyl lysine powder, so that the modified pearlescent powder can be softer and more skin-adhesive. Meanwhile, the modified pearlescent powder can have an extremely low surface tension, and a hydrophobic and lipophobic effect.

The present disclosure further provides a method of preparing the modified pearlescent powder as described above, which can include the following steps: mixing the pearlescent powder, the fluorine-containing silane coupling agent and the lauroyl lysine powder to obtain a mixture; and heating the mixture to obtain the modified pearlescent powder.

When the fluorine-containing silane coupling agent is exposed in the air, the alkoxy group thereof is hydrolyzed and forms silanol. In one embodiment, the pearlescent powder and the lauroyl lysine powder can be firstly mixed to obtain a mixture, and then the fluorine-containing silane coupling can be added into the mixture, so as to prevent the fluorine-containing silane coupling agent from being connected to the surface of the pearlescent powder due to a premature hydrolysis of the fluorine-containing silane coupling agent, and affecting the coating effect of the lauroyl lysine powder on the surface of pearlescent powder.

Meanwhile, the lauroyl lysine powder can be firstly randomly coated on the surface of the pearlescent powder, and then the fluorine-containing silane coupling agent can react with uncoated surfaces of the pearlescent powder. Fluorine-containing segments of the fluorine-containing silane coupling agent can be modified on the pearlescent powder by the covalent bonds, such that the lauroyl lysine powder and the fluorine-containing segments of the fluorine-containing silane coupling agent can be alternately distributed with each other.

In one embodiment, a temperature of the heating is in a range of 100 degrees centigrade to 120 degrees centigrade, and a time of the heating is in a range of 1 hour to 3 hours. During the heating process, the alkoxy group of the fluorine-containing silane coupling agent can be hydrolyzed and form a silanol, the silanol can react with hydroxyl groups on the surface of the pearlescent powder to form hydrogen bonds, and the hydrogen bonds can be dehydrated and condensed to obtain the fluorine-containing silane coupling agent. Meanwhile, the lauroyl lysine powder can be coated on the surface of the pearlescent powder via the physical effect of van der Waals forces.

Therefore, in the present disclosure, the modified pearlescent powder that is softer and more skin-adhesive while having a hydrophobic and lipophobic effect can be obtained by a dry process. The dry process is simpler and more suitable for industrial production.

In the process of mixing the pearlescent powder, the fluorine-containing silane coupling agent and the lauroyl lysine powder, the layered pearlescent powder will inevitably be broken into pearlescent powders having smaller particle sizes. Accordingly, the pearlescent effect, the color effect, and the glittery effect of the layered pearlescent powder will be weakened. However, compared to the pearlescent powder before mixing, the pearlescent effect, the color effect, and the glittery effect of the modified pearlescent powder obtained by modification with the fluorine-containing silane coupling agent and the lauroyl lysine powder can be improved.

The present disclosure can further provide a cosmetic including the modified pearlescent powder as described above.

Because the modified pearlescent powder has an extremely low surface tension and a hydrophobic and lipophobic effect, the spreadability of cosmetics including the modified pearlescent powder may be effectively improved, such that the cosmetics may be applied more evenly on the skin, and the makeup retention thereof can be better.

Meanwhile, the modified pearlescent powder may be directionally distributed in the cosmetic and form a mirror effect, such that the cosmetic exhibits higher glossiness.

It should be noted that when the modified pearlescent powder is directionally distributed in the cosmetics, it does not mean that the arrangement angles of the modified pearlescent powder are completely consistent, but tend to be consistent.

In one embodiment, the cosmetics can include pressed powder products, water products, oil products, emulsified products, and the like.

Taking a liquid eye shadow as an example, the liquid eye shadow can include the modified pearlescent powder as described above, and the mass percentage of the modified pearlescent powder in the liquid eye shadow is in a range of 1% to 40%, optionally in a range of 10% to 20 %.

In addition, the liquid eye shadow further can include water in a range of 35% to 90%, polyol in a range of less than or equal to 20%, a thickener in a range of less than or equal to 2%, an emulsifier in a range of less than or equal to 2%, a preservative in a range of 0.5% to 1%, and a membrane-forming agent in a range of less than or equal to 2% by mass.

The polyol can include at least one of glycerin, propylene glycol, or butylene glycol. The thickener can include at least one of xanthan gum, carbomer, hectorite, aluminum magnesium silicate, or cellulose. The emulsifier can include at least one of polysorbate-80, polysorbate-60, PEG-80 sorbitan laurate, or PEG-7 glycerol cocoate. The preservative can include at least one of a mixture of phenoxyethanol and ethylhexylglycerin, a mixture of caprylyl glycol and ethylhexylglycerin, or ethylhexylglycerin. The membrane-forming agent can include at least one of polyurethanes or polyacrylate copolymers.

Since the modified pearlescent powder has an extremely low surface tension and a hydrophobic and lipophobic effect, the overall surface energy of the liquid eye shadow can be reduced, so that the liquid eye shadow can have better spreadability and film-forming properties during application, and the liquid eye shadow can have better makeup retention.

Meanwhile, the modified pearlescent powder may be directionally distributed in the liquid eye shadow and form a mirror effect. Therefore, the liquid eye shadow may exhibit higher glossiness.

Hereinafter, the modified pearlescent powder, the preparation method thereof, and the cosmetics will be further described with reference to the following embodiments.

### Embodiment 1

30g of lauroyl lysine powder in a flake-shaped structure and 1kg of pearlescent powder (having particle sizes in a range of 10 µm to 60 µm, in a color of silver white, a substrate of mica, and a coating layer of titanium dioxide) were mixed evenly, and then 10g of perfluorooctyltriethoxysilane was added thereto and mixed evenly to obtain a mixture. The mixture was heated to 110 degrees centigrade and kept for 2 hours to obtain a modified pearlescent powder.

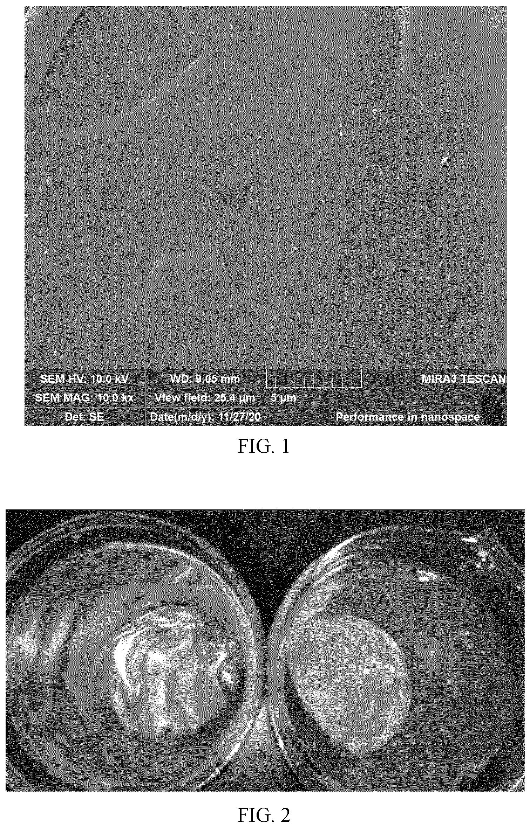

As shown in FIG. 1, in the modified pearlescent powder obtained in this embodiment, the lauroyl lysine powder having the flake-shaped structure was covered on a surface of the pearlescent powder. In addition, in the modified pearlescent powder, the fluorine-containing silane coupling agent was connected to the pearlescent powder via covalent bonds, and parts of the fluorine-containing silane coupling agent associated with each other and formed a film with a network structure, wherein the lauroyl lysine powder in the flake-shaped structure and the fluorine-containing silane coupling agent were distributed alternately with each other.

The pearlescent powder before modification and the obtained modified pearlescent powder of this embodiment were dispersed in glycerin. As shown in FIG. 2, the left side of the image showed the dispersion effect of the modified pearlescent powder, and the right side of the image showed the dispersion effect of the pearlescent powder before modification. It can be concluded from FIG. 2 that, because the modified pearlescent powder may be directionally distributed in glycerin and form a mirror effect, the modified pearlescent powder that was dispersed in the glycerin at the left side of the image exhibited higher gloss.

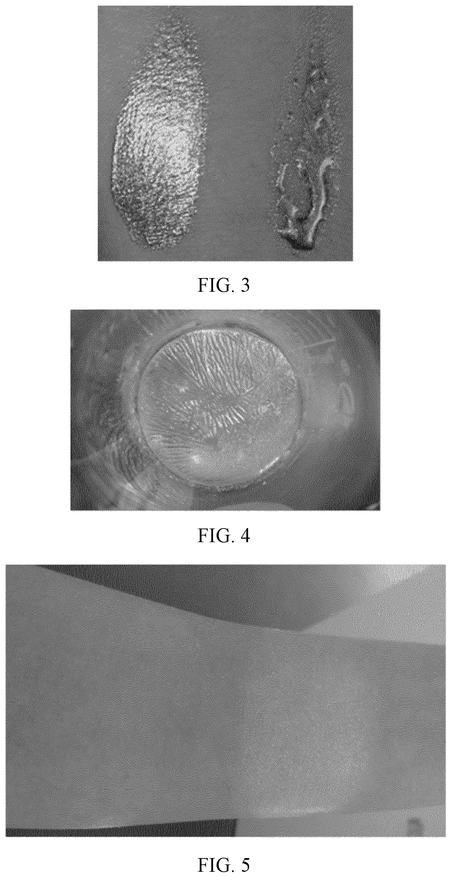

The modified pearlescent powder which was dispersed in the glycerin and the pearlescent powder before modification which was dispersed in glycerin were applied on the skin. As shown in FIG. 3, the left side of the image showed an applying effect of the modified pearlescent powder, and the right side of the image showed an applying effect of the pearlescent powder before modification. It can be concluded from FIG. 3 that, since the modified pearlescent powder had an extremely low surface tension and a hydrophobic and lipophobic effect, but the pearlescent powder before modification was hydrophilic and lipophilic, the modified pearlescent powder which was dispersed in glycerin may exhibit better spreadability when applied to the skin, while the pearlescent powder before modification which was dispersed in glycerin shrunk.

The modified pearlescent powder obtained in this embodiment was dispersed in water. As shown in FIG. 4, the modified pearlescent powder had a relatively lower surface energy, and it tended to be aligned on the surface of the polar system and formed a mirror effect, and exhibited a relatively brighter metallic luster.

### Embodiment 2

Embodiment 2 differed from Embodiment 1 only in that particle sizes of the pearlescent powder in Embodiment 2 were in a range of 30 µm to 150 µm.

### Embodiment 3

Embodiment 3 differed from Embodiment 2 only in that a substrate of the pearlescent powder of Embodiment 3 was a glass substrate.

### Embodiment 4

Embodiment 4 differed from Embodiment 1 only in that the covering layer of the pearlescent powder of Embodiment 4 was iron oxide and titanium dioxide, and the color of the pearlescent powder was golden.

### Embodiment 5

Embodiment 5 differed from Embodiment 1 only in that the covering layer of the pearlescent powder of Embodiment 5 was iron oxide, and the color of the pearlescent powder was reddish brown.

### Embodiment 6

Embodiment 6 differed from Embodiment 1 only in that the covering layer of the pearlescent powder of Embodiment 6 was organic lake and titanium dioxide, and the color of the pearlescent powder was rose red.

### Embodiment 7

Embodiment 7 differed from Embodiment 1 only in that the mass of the lauroyl lysine powder with the flake-shaped structure in Embodiment 7 was 1g.

### Embodiment 8

Embodiment 8 differed from Embodiment 1 only in that the mass of the lauroyl lysine powder with the flake-shaped structure in Embodiment 8 was 100g.

### Embodiment 9

Embodiment 9 differed from Embodiment 1 only in that the mass of the perfluorooctyltriethoxysilane in Embodiment 9 was 1 g.

### Embodiment 10

Embodiment 10 differed from Embodiment 1 only in that the mass of the perfluorooctyltriethoxysilane in Embodiment 10 was 100 g.

### Comparative Embodiment 1

Comparative Embodiment 1 differed from Embodiment 1 only in that the perfluorooctyltriethoxysilane was not added during the mixing process of Comparative Embodiment 1.

### Comparative Embodiment 2

Comparative Embodiment 2 differed from Embodiment 1 only in that the lauroyl lysine powder was not added during the mixing process of Comparative Embodiment 2.

The pearlescent powder before modification of each Embodiment and the modified pearlescent powder obtained in each Embodiment and Comparative Embodiment were dispersed in water and white mineral oil to determine the dispersibility. The results were shown in Table 1.

**Table 1**

| | Pearlescent powder before modification | | | Modified pearlescent powder | | |
|---|---|---|---|---|---|---|
| | Dispersion state in water | Dispersion state in white mineral oil | Dispersibility | Dispersed state in water | Dispersed state in white mineral oil | Dispersibility |
| Embodiment 1 | Dispersed | Dispersed | Hydrophilic and lipophilic | Floating on the surface of water | Agglomera ted | Hydrophobic and lipophobic |
| Embodiment 2 | Dispersed | Dispersed | Hydrophilic and lipophilic | Floating on the surface of water | Agglomera ted | Hydrophobic and lipophobic |
| Embodiment 3 | Dispersed | Dispersed | Hydrophilic and lipophilic | Floating on the surface of water | Agglomera ted | Hydrophobic and lipophobic |
| Embodiment 4 | Dispersed | Dispersed | Hydrophilic and lipophilic | Floating on the surface of water | Agglomera ted | Hydrophobic and lipophobic |
| Embodiment 5 | Dispersed | Dispersed | Hydrophilic and lipophilic | Floating on the surface of water | Agglomera ted | Hydrophobic and lipophobic |
| Embodiment 6 | Dispersed | Dispersed | Hydrophilic and lipophilic | Floating on the surface of water | Agglomera ted | Hydrophobic and lipophobic |
| Embodiment 7 | Dispersed | Dispersed | Hydrophilic and lipophilic | Floating on the surface of water | Agglomera ted | Hydrophobic and lipophobic |
| Embodiment 8 | Dispersed | Dispersed | Hydrophilic and lipophilic | Floating on the surface of water | Agglomera ted | Hydrophobic and lipophobic |
| Embodiment 9 | Dispersed | Dispersed | Hydrophilic and lipophilic | Floating on the surface of water | Agglomera ted | Hydrophobic and lipophobic |
| Embodiment 10 | Dispersed | Dispersed | Hydrophilic and lipophilic | Floating on the surface of water | Agglomera ted | Hydrophobic and lipophobic |
| Comparative Embodiment 1 | Dispersed | Dispersed | Hydrophilic and lipophilic | Partly floating on the surface of water | Dispersed | Slightly hydrophobic and lipophilic |
| Comparative Embodiment 2 | Dispersed | Dispersed | Hydrophilic and lipophilic | Floating on the surface of water | Agglomera ted | Hydrophobic and lipophobic |

### Application Embodiment 1-1

An eye shadow was provided. The eye shadow included a phase A, a phase B, and a phase C. The phase A included mica, talc powder, silica, methyl methacrylate cross-linked polymer, magnesium myristate, and magnesium stearate. The phase B included polydimethylsiloxane, caprylic/capric triglyceride, isononyl isononanoate, VP/hexadecene copolymer. The phase C was the modified pearlescent powder of Embodiment 1. Based on a total mass of 100%, a mass percentage of the mica was 50%, a mass percentage of the talc powder was 20%, a mass percentage of the silica was 5%, a mass percentage of the methyl methacrylate cross-linked polymer was 3%, a mass percentage of the magnesium myristate was 1%, a mass percentage of the magnesium stearate was 1%, a mass percentage of the polydimethylsiloxane was 3%, a mass percentage of the caprylic/capric triglyceride was 2%, a mass percentage of the isononyl isononanoate was 2%, a mass percentage of the VP/hexadecene copolymer was 1%, and a mass percentage of the modified pearlescent powder was 12%.

During preparation, firstly, the phase A was added into a stirring pot and was stirred evenly at a high speed. Then, the phase B was mixed and then added into the phase A, and continuously stirred evenly at a high speed. Thereafter, the phase C was added into the well-mixed phases A and B, and was stirred evenly at a low speed to obtain a mixture. Finally, the mixture was discharged and pressed into shape to obtain the eye shadow.

### Application Embodiment 1-2 to Application Embodiment 1-6

The compositions and mass percentages of the eye shadows of Application Embodiment 1-2 to Application Embodiment 1-6 differed from those of the eye shadows of Application Embodiment 1-1 in that the modified pearlescent powders of Embodiments 2-6 were used as the phases C of Application Embodiment 1-2 to Application Embodiment 1-6, respectively.

### Application Comparative Embodiment 1-1 to Application Comparative Embodiment 1-6

The compositions and mass percentages of the eye shadows of Application Comparative Embodiment 1-1 to Application Comparative Embodiment 1-6 differed from the eye shadows of Application Embodiment 1-1 in that the pearlescent powders of Embodiment 1 to Embodiment 6 were used for phases C, respectively.

### Application Comparative Embodiment 1-7 and Application Comparative Embodiment 1-8

The compositions and mass percentages of the eye shadows of Application Comparative Embodiment 1-7 and Application Comparative Embodiment 1-8 differed from those of the eye shadows of Application Embodiment 1-1 in that the modified pearlescent powders of Comparative Embodiment 1 and Comparative Embodiment 2 were used as the phases C of Application Comparative Embodiment 1-7 and Application Comparative Embodiment 1-8, respectively.

The eye shadows obtained in Application Embodiment 1-1 and Application Comparative Embodiment 1-1 were applied to the skin, and the results were shown in FIG. 5. The right side of the image in FIG. 5 was the application effect of the eye shadow of Application Embodiment 1-1, and the left side of the image in FIG. 5 was the application effect of the eye shadow of Application Comparative Embodiment 1-1. It can be concluded from FIG. 5 that the eye shadow including the modified pearlescent powder of the present disclosure had a better spreadability, a better adhesion to the skin and better makeup retention.

The eye shadows of Application Embodiment 1-1 to Application Embodiment 1-6 and Application Comparative Embodiment 1-1 to Application Comparative Embodiment 1-8 were tested in terms of glossiness, skin softness and makeup retention. The results were shown in Table 2, wherein when evaluated on a scale of 1 to 10 points, the higher the score was, the higher the gloss, the better the softness and skin-adhesion, and the better the makeup retention of the eye shadow were.

**Table 2**

| | Glossiness | Softness and skin-adhesion | Makeup retention |
|---|---|---|---|
| Application Embodiment 1-1 | 7 | 9 | 10 |
| Application Embodiment 1-2 | 8 | 8 | 8 |
| Application Embodiment 1-3 | 10 | 7 | 8 |
| Application Embodiment 1-4 | 7 | 9 | 10 |
| Application Embodiment 1-5 | 7 | 9 | 10 |
| Application Embodiment 1-6 | 7 | 9 | 10 |
| Application Comparative Embodiment 1-1 | 3 | 4 | 5 |
| Application Comparative Embodiment 1-2 | 4 | 3 | 4 |
| Application Comparative Embodiment 1-3 | 5 | 2 | 4 |
| Application Comparative Embodiment 1-4 | 3 | 4 | 5 |
| Application Comparative Embodiment 1-5 | 3 | 4 | 5 |
| Application Comparative Embodiment 1-6 | 3 | 4 | 5 |
| Application Comparative Embodiment 1-7 | 3 | 7 | 6 |
| Application Comparative Embodiment 1-8 | 7 | 5 | 10 |

### Application Embodiment 2-1

A liquid eye shadow was provided. The included deionized water, glycerin, xanthan gum, O/W emulsifier, polyurethane, and the modified pearlescent powder of Embodiment 1. Based on a total mass of 100%, a mass percentage of the deionized water was 67.5%, a mass percentage of the glycerin was 10%, a mass percentage of the xanthan gum was 0.5%, a mass percentage liquid eye shadow of the O/W emulsifier was 1.5%, a mass percentage of the modified pearlescent powder of Embodiment 1 was 20%, and a mass percentage of the polyurethane was 0.5%.

### Application Embodiment 2-2 to Application Embodiment 2-6

The compositions and mass percentages of the liquid eye shadows of Application Embodiment 2-2 to Application Embodiment 2-6 differed from those of the liquid eye shadows of Application Embodiment 2-1 in that the modified pearlescent powders of Embodiment 2 to Embodiment 6 were used in Application Embodiment 2-2 to Application Embodiment 2-6, respectively.

### Application Comparative Embodiment 2-1 to Application Comparative Embodiment 2-6

The compositions and mass percentages of the liquid eye shadows of Application Comparative Embodiment 2-1 to Application Comparative Embodiment 2-6 differed from those of the liquid eye shadows of Application Embodiment 2-1 in that the pearlescent powders of Embodiment 1 to Embodiment 6 were used in Comparative Embodiment 2-1 to Comparative Embodiment 2-6, respectively.

### Application Comparative Embodiment 2-7 and Application Comparative Embodiment 2-8

The compositions and mass percentages of the liquid eye shadows of Application Comparative Embodiment 2-7 and Application Comparative Embodiment 2-8 differed from those of the liquid eye shadows of Application Embodiment 2-1 in that the modified pearlescent powders of Comparative Embodiment 1 and Comparative Embodiment 2 were used in Application Comparative Embodiment 2-7 and Application Comparative Embodiment 2-8, respectively.

The liquid eye shadows of Application Embodiments 2 to Application Embodiment 6 and Application Comparative Embodiments 1 to Application Comparative Embodiment 8 were tested in terms of glossiness, softness and skin-adhesion, and makeup retention. The results were shown in Table 3, wherein when evaluated on a scale of 1 point to 10 points, the higher the score was, the higher the gloss, the better the softness and skin-adhesion, and the better the makeup retention of the liquid eye shadow were.

**Table 3**

| | Glossiness | Softness and skin-adhesion | Makeup retention |
|---|---|---|---|
| Application Embodiment 2-1 | 9 | 9 | 10 |
| Application Embodiment 2-2 | 9 | 8 | 8 |
| Application Embodiment 2-3 | 10 | 7 | 7 |
| Application Embodiment 2-4 | 9 | 9 | 10 |
| Application Embodiment 2-5 | 9 | 9 | 10 |
| Application Embodiment 2-6 | 9 | 9 | 10 |
| Application Comparative Embodiment 2-1 | 5 | Did not form a film after application, and shrunk immediately | |
| Application Comparative Embodiment 2-2 | 5 | | |
| Application Comparative Embodiment 2-3 | 8 | | |
| Application Comparative Embodiment 2-4 | 5 | | |
| Application Comparative Embodiment 2-5 | 5 | | |
| Application Comparative Embodiment 2-6 | 5 | | |
| Application Comparative Embodiment 2-7 | 5 | Did not form a film after application, and shrunk immediately | |
| Application Comparative Embodiment 2-8 | 9 | 5 | 10 |

### Application Embodiment 3-1

A liquid highlighter was provided. This product included a phase A, a phase B, and a phase C. The phase A included polydimethylsiloxane, isododecane, cyclopentasiloxane, disteardimonium hectorite, phenyl trimethicone, PEG-10 dimethicone, caprylic/capric triglyceride, trimethylsiloxysilicate, and polymethylsilsesquioxane. The phase B included water and alcohol. The phase C was the modified pearlescent powder of Embodiment 1. Based on a total mass of 100 %, a mass percentage of the polydimethylsiloxane was 9%, a mass percentage of the isododecane was 14.3 %, a mass percentage of the cyclopentasiloxane was 20%, a mass percentage of the disteardimonium hectorite was 0.7%, a mass percentage of the phenyl trimethicone was 6%, a mass percentage of the PEG-10 dimethicone was 3%, a mass percentage of the caprylic/capric triglyceride was 5%, a mass percentage of the trimethylsiloxysilicate was 2%, a mass percentage of the polymethylsilsesquioxane was 5%, a mass percentage of the water was 5%, a mass percentage of the alcohol was 12%, and a mass percentage of the modified pearlescent powder was 18%.

During preparation, firstly, the phase A was added into a beaker and stirred evenly at a medium speed. Then, the phase B was mixed and added into the phase A, and homogenized for 10 minutes. Thereafter, the phase C was added into the well-mixed phase A and phase B, and the mixture was stirred evenly at a low speed, thereby obtaining a liquid highlighter.

### Application Embodiment 3-2 to Application Embodiment 3-6

The compositions and mass percentages of the liquid highlighters of Application Embodiment 3-2 to Application Embodiment 3-6 differed from those of the liquid highlighters of Application Embodiment 3-1 in that the modified pearlescent powders of Embodiment 2 to Embodiment 6 were used as phases C of Application Embodiment 3-2 to Application Embodiment 3-6, respectively.

### Application Comparative Embodiment 3-1 to Application Comparative Embodiment 3-6

The compositions and mass percentages of the liquid highlighters of Application Comparative Embodiment 3-1 to Application Comparative Embodiment 3-6 differed from those of the liquid highlighters of Application Embodiment 3-1 in that the pearlescent powders of Embodiment 1 to Embodiment 6 were used as phases C of Application Comparative Embodiment 3-1 to Application Comparative Embodiment 3-6, respectively.

### Application Comparative Embodiment 3-7 and Application Comparative Embodiment 3-8

The compositions and mass percentages of the liquid highlighters of Application Comparative Embodiment 3-7 and Application Comparative Embodiment 3-8 differed from those of the liquid highlighters of Application Embodiment 3-1 in that the modified pearlescent powders of Comparative Embodiment 1 and Comparative Embodiment 2 were used as the phases C of Application Comparative Embodiment 3-7 and Application Comparative Embodiment 3-8, respectively.

The liquid highlighter of Application Embodiment 3-1 to Application Embodiment 3-6 and Application Comparative Embodiment 3-1 to Application Comparative Embodiment 3-8 were tested in terms of glossiness, softness and skin-adhesion, and makeup retention. The results were shown in Table 4, wherein when evaluated on a scale of 1 to 10, the higher the score, the higher the gloss, the better the softness and skin-adhesion, and the better the makeup retention of the liquid highlighter were.

**Table 4**

| | Glossiness | Softness and skin-adhesion | Makeup retention |
|---|---|---|---|
| Application Embodiment 3-1 | 7 | 9 | 10 |
| Application Embodiment 3-2 | 8 | 8 | 8 |
| Application Embodiment 3-3 | 10 | 7 | 8 |
| Application Embodiment 3-4 | 7 | 9 | 10 |
| Application Embodiment 3-5 | 7 | 9 | 10 |
| Application Embodiment 3-6 | 7 | 9 | 10 |
| Application Comparative Embodiment 3-1 | 6 | 5 | 6 |
| Application Comparative Embodiment 3-2 | 7 | 4 | 5 |
| Application Comparative Embodiment 3-3 | 8 | 3 | 5 |
| Application Comparative Embodiment 3-4 | 6 | 5 | 6 |
| Application Comparative Embodiment 3-5 | 6 | 5 | 6 |
| Application Comparative Embodiment 3-6 | 6 | 5 | 6 |
| Application Comparative Embodiment 3-7 | 3 | 7 | 7 |
| Application Comparative Embodiment 3-8 | 7 | 6 | 10 |

### Application Embodiment 4-1

A liquid highlighter (W/O type) was provided: the W/O type liquid highlighter was an emulsified product included a phase A, a phase B, a phase C and a phase D. The phase A included PEG-10 dimethicone and lauryl PEG-9 poly dimethylsiloxyethyl dimethicone, phenyl trimethicone, caprylic/capric triglyceride, octyldodecanol, butylene glycol dicaprylate/dicaprate, trimethylsiloxysilicate and/or polydimethylsiloxane, and a C9-12 alkane. The phase B included cyclopentadimethylsiloxane and disteardimonium hectorite. The phase C included water, glycerin, magnesium sulfate, propylene glycol, and phenoxyethanol/ethylhexylglycerin. The phase D was the modified pearlescent powder of Embodiment 1. Based on a total mass of 100%, a mass percentage of the PEG-10 dimethicone was 2%, a mass percentage of the lauryl PEG-9 poly dimethylsiloxyethyl dimethicone was 1%, a mass percentage of the phenyl trimethicone was 7%, a mass percentage of the caprylic /capric triglyceride was 4%, a mass percentage of the octyldodecanol was 5%, a mass percentage of the butylene glycol dicaprylate/dicaprate was 5%, a mass percentage of the trimethylsiloxysilicate was 2%, a mass percentage of the C9-12 alkane was 6%, a mass percentage of the cyclopentadimethylsiloxane was 15%, a mass percentage of the disteardimonium hectorite was 1%, a mass percentage of the water was 30.55%, a mass percentage of the glycerin was 5%, a mass percentage of the magnesium sulfate was 0.7%, a mass percentage of the propylene glycol was 5%, a mass percentage of the phenoxyethanol/ethylhexyl glycerin was 0.75%, and a mass percentage of the modified pearlescent powder was 10%.

During preparation, firstly, the phase A was added into a beaker, and was stirred and dispersed evenly at 60 degrees centigrade. Then, the phase B was mixed and added into phase A, and homogenized for 5 minutes. While homogenizing, the phase C was added into the homogenized phase A and phase B to obtain a mixture, and the mixture was continuously homogenized for 5 minutes after phase C was completely added. Thereafter, the phase D was added into the well-mixed phase A, phase B and phase C and the resultant was stirred evenly at low speed, thereby obtaining the liquid highlighter (W/O type).

### Application Embodiment 4-2 to Application Embodiment 4-6

The compositions and mass percentages of the liquid highlighters of Application Embodiment 4-2 to Application Embodiment 4-6 differed from those of the liquid highlighters of Application Embodiment 4-1 in that the modified pearlescent powders of Embodiment 2 to Embodiment 6 were used as the phase D of Application Embodiment 4-2 to Application Embodiment 4-6, respectively.

### Application Comparative Embodiment 4-1 to Application Comparative Embodiment 4-6

The compositions and mass percentages of the liquid highlighters of Application Comparative Embodiment 4-1 to Application Comparative Embodiment 4-6 differed from those of the liquid highlighters of Application Embodiment 4-1 in that the modified pearlescent powders of Embodiment 1 to Embodiment 6 were used as phases D of Application Embodiment 4-1 to Application Embodiment 4-6, respectively.

### Application Comparative Embodiments 4-7 and Application Comparative Embodiment 4-8

The compositions and mass percentages of the liquid highlighters of Application Comparative Embodiments 4-7 to 4-8 differed from those of the liquid highlighters of Application Embodiment 3-1 in that the modified pearlescent powders of Comparative Embodiment 1 and Comparative Embodiment 2 were used as the phases C of Application Comparative Embodiment 4-7 and Application Comparative Embodiment 4-8.

The liquid highlighter (W/O type) of Application Embodiment 4-1 to Application Embodiment 4-6 and Application Comparative Embodiment 4-1 to Application Comparative Embodiment 4-8 were tested in terms of glossiness, softness and skin-adhesion, and makeup retention. The results are shown in Table 5, wherein when evaluated on a scale of 1 to 10 points, the higher the score was, the higher the glossiness, the better the softness and skin-adhesion, and the better the makeup retention of the liquid highlighter (W/O type) were.

**Table 5**

| | Glossiness | Softness and skin-adhesion | Makeup retention |
|---|---|---|---|
| Application Embodiment 4-1 | 7 | 9 | 10 |
| Application Embodiment 4-2 | 8 | 8 | 8 |
| Application Embodiment 4-3 | 10 | 7 | 8 |
| Application Embodiment 4-4 | 7 | 9 | 10 |
| Application Embodiment 4-5 | 7 | 9 | 10 |
| Application Embodiment 4-6 | 7 | 9 | 10 |
| Application Comparative Embodiment 4-1 | 6 | 5 | 6 |
| Application Comparative Embodiment 4-2 | 7 | 4 | 5 |
| Application Comparative Embodiment 4-3 | 8 | 3 | 5 |
| Application Comparative Embodiment 4-4 | 6 | 5 | 6 |
| Application Comparative Embodiment 4-5 | 6 | 5 | 6 |
| Application Comparative Embodiment 4-6 | 6 | 5 | 6 |
| Application Comparative Embodiment 4-7 | 3 | 7 | 7 |
| Application Comparative Embodiment 4-8 | 7 | 6 | 10 |

The technical features of the above-described embodiments may be combined in any combination. For the sake of brevity of description, not all possible combinations of the technical features in the above embodiments are described. However, as long as there is no contradiction between the combinations of these technical features, all should be considered as within the scope of this disclosure.

The above-described embodiments are merely illustrative of several embodiments of the present disclosure, and the description thereof is relatively specific and detailed, but is not to be construed as limiting the scope of the disclosure. It should be noted that a number of variations and modifications may be made by those skilled in the art without departing from the spirit and scope of the disclosure. Therefore, the scope of the disclosure should be determined by the appended claims.

## Claims

1. A modified pearlescent powder comprising a pearlescent powder, a lauroyl lysine powder covered on a surface of the pearlescent powder, and a fluorine-containing silane coupling agent connected to the pearlescent powder by covalent bonds.

2. The modified pearlescent powder of claim 1, wherein the lauroyl lysine powder is in a flake-shaped structure.

3. The modified pearlescent powder of claim 1, wherein a part of the fluorine-containing silane coupling agent is associated with each other to form a network structure.

4. The modified pearlescent powder of claim 1, wherein a mass of the fluorine-containing silane coupling agent is in a range of 0.1% to 10.0% of a mass of the pearlescent powder, and a mass of the lauroyl lysine powder is in a range of 0.1% to 10.0% of the mass of the pearlescent powder.

5. The modified pearlescent powder of claim 1, wherein a general formula of the fluorine-containing silane coupling agent is as follows: R-Si-(OCₘH₂ₘ₊₁)₃, wherein R is defined as CₙF₂ₙ₊₁-(CH₂)₂-, n is an integer which is greater than or equal to 1 and less than or equal to 6, and m is 1 or 2.

6. The modified pearlescent powder of claim 5, wherein the fluorine-containing silane coupling agent comprises at least one of perfluorooctyltriethoxysilane, tridecafluorooctyltrimethoxysilane, or heptadecafluorodecyltrimethoxysilane.

7. A method of preparing the modified pearlescent powder of any one of claims 1 to 6, comprising:
mixing the pearlescent powder, the fluorine-containing silane coupling agent and the lauroyl lysine powder to obtain a mixture; and
heating the mixture to obtain the modified pearlescent powder.

8. The method of claim 7, wherein a temperature of the heating is in a range of 100 degrees centigrade to 120 degrees centigrade, and a time of the heating is in a range of 1 hour to 3 hours.

9. A cosmetic comprising the modified pearlescent powder according to any one of claims 1 to 6.

10. The cosmetic of claim 9, wherein the modified pearlescent powder is directionally distributed in the cosmetic.
